# EUROPEAN PATENT APPLICATION

(11) **EP 4 617 360 A1**
(43) Date of publication of application: **17.09.2025**
(21) Application number: 24764105.3
(22) Date of filing: 06.02.2024
(51) Int. Cl.: C12N 1/20, C12P 19/32, C12N 15/01, C12R 1/15

(54) **STRAIN FOR PRODUCING 5'-GUANYLIC ACID, AND METHOD FOR PRODUCING 5'-GUANYLIC ACID USING SAME**

(30) Priority: 28.02.2023 KR 20230026877
(71) Applicant: CJ CheilJedang Corporation, Seoul 04560 (KR)
(72) Inventor: KIM, Dae Young, Seoul 04560 (KR); LEE, Ji Hyun, Seoul 04560 (KR); BAE, Hyun-jung, Seoul 04560 (KR); KIM, Doyeon, Seoul 04560 (KR); KIM, Sang Jun, Seoul 04560 (KR); CHOI, Seung-Dae, Seoul 04560 (KR)
(74) Representative: Meissner Bolte Partnerschaft mbB
(86) International application number: PCT/KR2024/001705
(87) International publication number: WO 2024/181705

(57) **Abstract**

The present disclosure relates to a *Corynebacterium ammoniagenes* CJG0497 strain deposited with Accession No. KCCM13320P; a method for producing 5'-guanosine monophosphate (GMP), comprising culturing the strain in a medium; a composition for producing 5'-guanosine monophosphate (GMP), comprising the strain, a culture product of the strain, a fermented product of the strain, or a combination of two or more thereof; and use of the strain for the production of 5'-guanosine monophosphate (GMP).

## Description

### [Technical Field]

The present disclosure relates to a *Corynebacterium ammoniagenes* CJG0497 strain deposited with Accession No. KCCM13320P; a method for producing 5'-guanosine monophosphate (GMP), including culturing the strain in a medium; a composition for producing 5'-guanosine monophosphate (GMP), including the strain, a culture product of the strain, a fermented product of the strain, or a combination of two or more thereof; and use of the strain for the production of 5'-guanosine monophosphate (GMP).

### [Background Art]

5'-guanosine monophosphate (GMP), which is an intermediate of the nucleic acid biosynthetic metabolic system, has physiological importance in animals and plants, and is used in a wide spectrum of fields including food and pharmaceutical industries and for a variety of medical purposes. In particular, GMP is one of the nucleic acid-based seasonings that are attractive as flavor-enhancing seasoning agents due to their high flavor-enhancing effects for food when used in combination with monosodium glutamate (MSG).

Examples of the method for producing GMP may include: (1) a method in which yeast RNA is degraded with a microorganism-derived enzyme or chemically; (2) a method of directly producing a nucleotide by culturing a microorganism in a medium containing sugar, a nitrogen source and a phosphoric acid source, and; (3) a method of converting an intermediate for the nucleotide synthesis into a nucleotide chemically or enzymatically. Currently, the combination method of fermentation and chemical synthesis, and enzymatic conversion is widely employed for industrial purposes.

The combination method consists of a fermentation process which produces 5'-xanthosine monophosphate (XMP), an intermediate of the nucleic acid biosynthetic metabolic system, using microorganisms, and an enzyme reaction process which converts the fermentation product into GMP, and XMP-producing microorganisms and microorganisms capable of converting XMP to GMP are used. Therefore, for efficient production of GMP, GMP degradation by microorganisms involved in GMP production must be suppressed, and in particular, microorganisms used during the enzyme reaction process must have a high complex capacity to convert XMP to GMP.

Accordingly, various studies for developing microorganisms with high-efficiency production and technologies for fermentation processes are underway. For example, target-specific approaches, such as increasing the expression of genes encoding enzymes related to XMP or GMP biosynthesis, or deleting genes not necessary for its biosynthesis, are mainly used (EP 3722430 A1, US 2020-0347346 A1).

However, in order to efficiently increase GMP-producing ability, research on novel strains with a high GMP conversion ability and a low GMP degradation rate is still needed.

### [Disclosure]

### [Technical Problem]

The present inventors confirmed that the *Corynebacterium ammoniagenes* CJG0497 strain deposited with Accession No. KCCM13320P has a high GMP conversion ability and a low GMP degradation rate, and thus can produce 5'-guanosine monophosphate (GMP) with high yield, thereby completing the present disclosure.

### [Technical Solution]

It is one object of the present disclosure to provide a *Corynebacterium ammoniagenes* CJG0497 strain deposited with Accession No. KCCM13320P.

In one embodiment, the strain may have a 5'-guanosine monophosphate (GMP)-producing ability.

In another embodiment, the strain may have a conversion ability to convert from 5'-xanthosine monophosphate (XMP) to 5'-guanosine monophosphate (GMP).

It is another object of the present disclosure to provide a method for producing 5'-guanosine monophosphate (GMP), including culturing a *Corynebacterium ammoniagenes* CJG0497 strain deposited with Accession No. KCCM13320P in a medium.

In one embodiment, the method may further include recovering 5'-guanosine monophosphate (GMP) from the cultured strain, a culture product of the strain, a fermented product of the strain, or the culture medium.

It is still another object of the present disclosure to provide a composition for producing 5'-guanosine monophosphate (GMP), including a *Corynebacterium ammoniagenes* CJG0497 strain deposited with Accession No. KCCM13320P, a culture product of the strain, a fermented product of the strain, or a combination of two or more thereof.

It is yet another object of the present disclosure to provide the use of a *Corynebacterium ammoniagenes* CJG0497 strain deposited with Accession No. KCCM13320P for the production of 5'-guanosine monophosphate (GMP).

### [Advantageous Effects]

The *Corynebacterium ammoniagenes* CJG0497 strain deposited with Accession No. KCCM13320P of the present disclosure has a high GMP conversion ability and a low GMP degradation rate, and thus can produce 5'-guanosine monophosphate (GMP) with high yield. Therefore, it can be effectively used for the industrial production of GMP.

### [Detailed Description of Preferred Embodiments]

The present disclosure will be described in detail as follows. Meanwhile, each description and embodiment disclosed herein can be applied to other descriptions and embodiments, respectively. That is, all combinations of various elements disclosed herein fall within the scope of the present disclosure. Further, the scope of the present disclosure is not limited by the specific description described below.

Additionally, those of ordinary skill in the art may be able to recognize or confirm, using only conventional experimentation, many equivalents to the particular aspects of the invention described herein. Furthermore, it is also intended that these equivalents be included in the present disclosure.

As used in the specification and appended claims, the singular forms ("a", "an", and "the") include plural referents unless the context clearly states otherwise. Unless the context indicates otherwise, singular terms shall include the plural and plural terms shall include the singular. As used in the specification and appended claims, unless stated otherwise, the use of "or" may be used to include "and/or".

As used herein, the term "about" may be presented before a particular numerical value. The term "about" used herein includes not only the exact number recited after the term, but also a range that is near or close to that number. Considering the context in which the number is presented, it can be determined whether any number is close to or near the particular number presented. In one example, the term "about" may refer to a range from -10% to +10% of a numerical value. In another example, the term "about" may refer to a range from -5% to +5% of a given numerical value, but is not limited thereto.

As used herein, the term "comprising/including" means the presence of features, steps or components recited after the term, and does not exclude the presence or addition of one or more features, steps or components. The components or features recited after the term "comprising/including" herein may be essential or mandatory. However, in some embodiments, the term may further include any other or non-essential components or features.

One aspect of the present disclosure provides a *Corynebacterium ammoniagenes* CJG0497 strain deposited with Accession No. KCCM13320P.

The strain of the present disclosure may refer to a mutant strain obtained by mutation of its parent strain. Specifically, the strain of the present disclosure may include all strains capable of producing desired 5'-guanosine monophosphate (GMP) by mutating the *Corynebacterium ammoniagenes* ATCC6872 strain (currently called *Corynebacterium stationis* ATCC6872 strain).

In this regard, mutagenesis of microorganisms may be performed by a variety of methods widely known in the art, and either physical mutagenesis or chemical mutagenesis may be used. For example, examples of physical mutagens suitable for the present disclosure may include gamma radiations and ultraviolet radiations, but the physical mutagens are not limited thereto. Additionally, examples of chemical mutagens may include N-methyl-N'-nitro-N-nitrosoguanidine (NTG), diepoxybutane, ethyl methanesulfonate, mustard compounds, hydrazine, and nitrite, but the chemical mutagens are not limited thereto.

When mutagenesis is induced, a parent strain is affected by a mutagen at a condition that can leave a particular size of a surviving population of the parent strain. The condition varies with the type of the mutagen and depends on the amount of mutation being induced within the surviving population at a constant killing rate. For example, in the case of ultraviolet radiation, the killing rate can leave approximately 0.00001% to 20% of the starting population viable. In the case of NTG, the killing rate can leave approximately 10% to 50% of the starting population viable, and mutagenesis by nitrite can leave approximately 0.01% to 0.1% of the starting population viable, but the mutagens are not limited thereto.

In one embodiment, the strain may have a 5'-guanosine monophosphate (GMP)-producing ability.

In another embodiment, the strain may have a conversion ability to convert from 5'-xanthosine monophosphate (XMP) to 5'-guanosine monophosphate (GMP).

As used herein, the term "5'-guanosine monophosphate (GMP)" refers to a nucleotide having a structure in which a phosphate group forms an ester bond with a ribose of a guanosine molecule. The 5'-guanosine monophosphate may be used interchangeably with GMP. The GMP may be synthesized by adding ammonia molecules to 5'-xanthosine monophosphate (XMP) via 5'-guanylic acid biosynthase (GMP synthase). A method of preparing GMP from XMP and/or a means used in the method may be selected from known techniques.

As used herein, the term "5'-xanthosine monophosphate (XMP) refers to a nucleotide resulting from dehydrogenation of IMP, and may be used interchangeably with XMP. The XMP may be synthesized from IMP by inosine-5'-monophosphate dehydrogenase.

The *Corynebacterium ammoniagenes* CJG0497 strain deposited with Accession No. KCCM13320P of the present disclosure has a high GMP conversion ability and a low GMP degradation rate, and thus can produce 5'-guanosine monophosphate (GMP) with high yield. Therefore, it can be effectively used for the industrial production of GMP.

**In** one embodiment, the strain of the present disclosure may be a strain having an increased 5'-guanosine monophosphate (GMP)-producing ability compared to a parent strain, but is not limited thereto. **In** one example, the parent strain, which is the target strain for comparing the increase in the 5'-guanosine monophosphate (GMP)-producing ability, may be *Corynebacterium ammoniagenes* ATCC6872 strain, but is not limited thereto.

**In** another embodiment, the strain of the present disclosure may be a strain having an increased conversion ability to convert from 5'-xanthosine monophosphate (XMP) to 5'-guanosine monophosphate (GMP) compared to the parent strain, but is not limited thereto.

In still another embodiment, the strain of the present disclosure may be a strain that has a reduced degradation rate of 5'-guanosine monophosphate (GMP) compared to the parent strain, but is not limited thereto.

Meanwhile, the *Corynebacterium ammoniagenes* CJG0497 strain deposited with Accession No. KCCM13320P having a 5'-guanosine monophosphate (GMP)-producing ability may include the strain itself, and all of the strains having an enhanced 5'-guanosine monophosphate (GMP)-producing ability by increasing or decreasing the activity of genes related to the GMP production mechanism, or strains having an enhanced 5'-guanosine monophosphate (GMP)-producing ability by introducing or increasing the activity of a foreign gene.

Another aspect of the present disclosure provides a method for producing 5'-guanosine monophosphate (GMP), including culturing a *Corynebacterium ammoniagenes* CJG0497 strain deposited with Accession No. KCCM13320P in a medium.

As used herein, the term "cultivation" means that the strain of the present disclosure is grown under appropriately controlled environmental conditions. The cultivation process of the present disclosure may be performed in a suitable culture medium and culture conditions known in the art. Such a cultivation process may be easily adjusted for use by those skilled in the art according to the strain to be selected. Specifically, the cultivation may be a batch culture, a continuous culture, and/or a fed-batch culture, but is not limited thereto.

As used herein, the term "medium" refers to a mixture of materials which contains nutrient materials required for the cultivation of the strain of the present disclosure as a main ingredient, and it supplies nutrient materials and growth factors, along with water that is essential for survival and growth. Specifically, the medium and other culture conditions used for culturing the strain of the present disclosure may be any medium used for conventional cultivation of strains without any particular limitation. However, the strain of the present disclosure may be cultured under aerobic conditions in a conventional medium containing an appropriate carbon source, nitrogen source, phosphorus source, inorganic compound, amino acid, and/or vitamin, while adjusting temperature, pH, *etc.* For example, the culture medium for the strains of the genus *Corynebacterium* can be found in the literature ["Manual of Methods for General Bacteriology" by the American Society for Bacteriology (Washington D.C., USA, 1981)].

In the present disclosure, the carbon source may include carbohydrates, such as glucose, saccharose, lactose, fructose, sucrose, maltose, *etc.;* sugar alcohols, such as mannitol, sorbitol, *etc.;* organic acids, such as pyruvic acid, lactic acid, citric acid, *etc.;* amino acids, such as glutamic acid, methionine, lysine, *etc.* Additionally, the carbon source may include natural organic nutrients such as starch hydrolysate, molasses, blackstrap molasses, rice bran, cassava, sugar cane molasses, and corn steep liquor, *etc.* Specifically, carbohydrates such as glucose and sterilized pretreated molasses (i.e., molasses converted to reducing sugar) may be used, and in addition, various other carbon sources in an appropriate amount may be used without limitation. These carbon sources may be used alone or in a combination of two or more kinds, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extract, yeast extract, malt extract, corn steep liquor, casein hydrolysate, fish or a decomposition product thereof, defatted soybean cake or a decomposition product thereof, *etc.* These nitrogen sources may be used alone or in a combination of two or more kinds, but are not limited thereto.

The phosphorus source may include monopotassium phosphate, dipotassium phosphate, or corresponding sodium-containing salts, *etc.* Examples of the inorganic compounds may include sodium chloride, calcium chloride, iron chloride, magnesium sulfate, iron sulfate, manganese sulfate, calcium carbonate, *etc.* Additionally, amino acids, vitamins, and/or appropriate precursors, *etc.,* may be included. These constituting ingredients or precursors may be added to a medium in a batch or continuous manner, but these phosphorus sources are not limited thereto.

Additionally, the pH of the medium may be adjusted by adding a compound such as ammonium hydroxide, potassium hydroxide, ammonia, phosphoric acid, sulfuric acid, *etc.* during the cultivation of the strain of the present disclosure in an appropriate manner. In addition, bubble formation may be prevented during the cultivation using an antifoaming agent such as fatty acid polyglycol ester. Further, oxygen gas or a gas containing oxygen may be injected to the medium order to maintain aerobic conditions of the medium; or nitrogen gas, hydrogen gas, or carbon dioxide may be injected to maintain anaerobic or microaerobic conditions, without the injection of gas, but the gas is not limited thereto.

The temperature during the cultivation of the present disclosure may be in the range from 27°C to 37°C, specifically from 30°C to 33°C, and the cultivation may be continued for 20 hours to 120 hours, but is not limited thereto.

As used herein, the term "culture product" means a culture solution, a concentrated culture solution, a dried product of a culture solution, a culture filtrate, a concentrated culture filtrate, or a dried product of a culture filtrate obtained by culturing a specific strain in a culture medium, and means that the culture solution may include the specific strain while the culture filtrate does not substantially include the specific strain (in particular, it substantially means excluding a specific strain isolated by filtration, *etc.,* but does not mean that the strain is completely excluded from the filtrate). The formulation of the culture product is not limited, and may be, for example, a liquid, emulsion, or solid. Specifically, for the purpose of the present disclosure, the culture product may include 5'-guanosine monophosphate (GMP).

As used herein, the term "fermentation" means that strains are not putrefactive during the process of decomposing organic matter using their own enzymes. Fermentation reaction and decay reaction proceed by similar processes, but when decomposition produces useful substances, it is called fermentation, and when odorous or harmful substances are produced, it is called decay.

In the present disclosure, the method for obtaining a fermented product from the strain is not particularly limited, and may be obtained according to a method commonly used in the art or similar fields.

As used herein, the term "fermented product" may include not only the fermented material itself, but also all kinds of materials including fermented products produced from the strain, such as a culture medium of the strain in which the strain and the culture coexist, a fermented product produced from the culture medium, a fermented product obtained by filtering the strain from the culture medium, a fermented product obtained by sterilizing the strain from the culture medium and filtering the same, an extract obtained by extracting the fermented product or a culture medium containing the same, a diluted solution obtained by diluting the fermented product or an extract thereof, a concentrated solution, a dried product obtained by drying the fermented product or an extract thereof, and a lysate obtained by collecting and lysing the cells of the strain, *etc.*

In the method of the present disclosure, the cultivation of the strain may be performed by any culture conditions and culture methods known in the art. Such a cultivation process can be easily adjusted and used by those skilled in the art according to the selected strain.

The 5'-guanosine monophosphate (GMP) produced by the cultivation of the present disclosure may be released into the medium or remain in the cells.

In one embodiment, the method for producing 5'-guanosine monophosphate (GMP) of the present disclosure may further include a step of preparing the strain of the present disclosure, a step of preparing a medium for culturing the strain, or a combination thereof (regardless of the order, in any order), for example, prior to the culturing step.

The method for producing 5'-guanosine monophosphate (GMP) of the present disclosure may further include recovering the desired substances, specifically, 5'-guanosine monophosphate (GMP) from the cultured strain, a culture product of the strain, a fermented product of the strain or the culture medium. The recovering step may be further included after the culturing step.

In the recovering step, the desired 5'-guanosine monophosphate (GMP) may be collected using the method of culturing the strain of the present disclosure, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatographies such as molecular sieve chromatography (gel filtration), adsorption chromatography, ion exchange chromatography, affinity chromatography, *etc.,* HPLC or a combination thereof may be used, and the desired substance, specifically, 5'-guanosine monophosphate (GMP), can be recovered from the medium or the strains using suitable methods known in the art.

Further, the method for producing 5'-guanosine monophosphate (GMP) of the present disclosure may further include a purification step, which may be performed using an appropriate method known in the art. In one example, when the method for producing 5'-guanosine monophosphate (GMP) of the present disclosure includes both a recovering step and a purification step, the recovering step and the purification step may be performed continuously or intermittently regardless of the order or simultaneously, or may be integrated into one step, but the method is not limited thereto.

In the method of the present disclosure, the strain and 5'-guanosine monophosphate (GMP), *etc.* are as described in other aspects above.

Still another aspect of the present disclosure provides a composition for producing 5'-guanosine monophosphate (GMP), including a *Corynebacterium ammoniagenes* CJG0497 strain deposited with Accession No. KCCM13320P, a culture product of the strain, a fermented product of the strain, or a combination of two or more thereof.

The composition of the present disclosure may further include any suitable excipient commonly used in compositions for producing 5'-guanosine monophosphate (GMP), and such excipients may include, for example, preservatives, wetting agents, dispersing agents, suspending agents, buffers, stabilizers, or isotonic agents, *etc.,* but are not limited thereto.

In one embodiment, each component present in the composition of the present disclosure may be contained in a mycologically effective amount, or in an amount that can be appropriately present in the composition for production.

In the composition of the present disclosure, the strain and 5'-guanosine monophosphate (GMP), *etc.* are as described in other aspects above.

Yet another aspect of the present disclosure provides the use of a *Corynebacterium ammoniagenes* CJG0497 strain deposited with Accession No. KCCM13320P for the production of 5'-guanosine monophosphate (GMP).

Even another aspect of the present disclosure provides the use of a composition including a *Corynebacterium ammoniagenes* CJG0497 strain deposited with Accession No. KCCM13320P, a culture product of the strain, a fermented product of the strain, or a combination of two or more thereof for the production of 5'-guanosine monophosphate (GMP).

In the use of the present disclosure, the strain and 5'-guanosine monophosphate (GMP), *etc.* are as described in other aspects above.

### [Mode for Carrying Out the Invention]

Hereinafter, the present disclosure will be described in detail by way of Examples. However, these Examples are merely preferred Examples given for illustrative purposes, and thus, the scope of the present disclosure is not intended to be limited to or by these Examples. Meanwhile, technical features which are not described herein can be sufficiently understood and easily carried out by those skilled in the art in the technical field of the present disclosure or in a similar technical field.

### Example 1: Selection of Mutant Strains Through Artificial Mutagenesis

In order to obtain a microbial mutant strain having a GMP-producing ability, mutation was induced in the *Corynebacterium ammoniagenes* ATCC6872*.*

The *Corynebacterium ammoniagenes* ATCC6872 strain is currently called *Corynebacterium stationis* ATCC6872 strain, and is also used as *Corynebacterium ammoniagenes* ATCC6872.

Specifically, for mutagenesis, a physical method of gamma ray irradiation was applied. High-energy gamma rays emitted from Co-60, a source of radiation, flow along chromosomes and randomly trigger changes in the nucleotide sequences, such as nucleotide substitutions, deletions, insertions, *etc.* Since the level of nucleotide sequence change is proportional to the intensity of gamma ray irradiation, high-level gamma ray induces mutation at a high rate and at the same time increases the killing rate of the strain. In order to sufficiently secure a high-quality mutant library with a high diversity of high mutation rate, gamma-irradiation conditions were precedingly established for the corynebacteria strains targeted for gamma-irradiation. Gamma rays with an intensity of 0, 0.5, 1, 2, 3, 4, 5, 7.5, and 10-kGy/hr were each applied for 1 hour to 30 mL of a seed medium culture solution having an absorbance of 6.09 at 562 nm using a high-level gamma ray irradiation device of Nordion Inc. located at the Advanced Radiation Technology Institute of the Korea Atomic Energy Research Institute, 100 µl of each gamma-ray irradiation solution diluted at a dilution rate of 10⁰, 10⁻¹, 10⁻², 10⁻³, and 10⁻⁴ was plated on the seed medium, and the plate medium plated with the gamma-ray irradiation solution was incubated in a 30°C stationary incubator for 48 hours, and then the number of individual colonies produced was counted to calculate the killing rate for each irradiation condition. CFU/mL according to gamma irradiation conditions of 0, 0.5, 1, 2, 3, 4, 5, 7.5, and 10-kGy/hr was 3.6X10⁸, 6.9X10⁷, 9.8X10⁶, 2.7X10⁵, 2.1X10³, 3.0X10¹, 0, 0, and 0, respectively, and the killing rates calculated therefrom were 0, 81.00467, 97.31540, 99.92589, 99.99942, 99.99999, 100, 100, and 100%, respectively. Based on the above irradiation conditions, it was confirmed that the gamma irradiation condition for securing a high-quality mutant library with a high mutational diversity through corynebacteria strain-based gamma irradiation was found to be 4-kGy/hr.

In order to obtain microorganisms with a high GMP conversion ability, a gamma-irradiation-based mutant library was constructed based on the ATCC6872 strain, and mutants with a high GMP conversion ability were screened. For gamma irradiation of the ATCC6872 strain, a 500 mL flask containing 50 mL of the seed medium was incubated for 24 hours in a shaking incubator at 30°C to obtain a culture solution with an Absorbance of 7.84 at 562 nm, and then diluted to an Absorbance of 6.10 at 562 nm using the seed medium. After irradiating 4 kGy/hr of gamma rays to 30 mL of the diluted solution for 1 hour using a high-level gamma ray irradiation device of the Advanced Radiation Technology Institute of the Korea Atomic Energy Research Institute, 100 µl of the irradiated stock solution was plated on 100 Petri dishes (90 X 15 mm) of the seed medium. Then, a total of about 40,000 individual colonies were secured by incubating the Petri dishes in a 30°C stationary incubator for 48 hours, and in order to secure the stability of the mutants, all individual colonies generated in the Petri dishes were recovered and suspended in a 20% glycerol solution, and then subdivided into 1 mL in 1.5 mL microtubes and stored in a cryogenic freezer at -80°C. When screening the mutants with a high GMP conversion ability, a 20% glycerol suspension solution thawed at room temperature, which was stored in a cryogenic freezer, was serially diluted to 10⁻¹, 10⁻², 10⁻³, 10⁻⁴, and 10⁻⁵ using saline. Then, 100 µl of the diluted solution corresponding to each dilution factor was plated in the seed medium, and reactivated in the form of individual colonies by incubating for 24 hours in a 30°C stationary incubator. The reactivated individual colonies were inoculated into a 96-deep well plate containing 350 µl of the seed medium with a filling rate of 17% per well using Qpix 420, a Colony Picker of Molecular Devices, and the ATCC6872 strain, which was not irradiated with gamma rays, was inoculated into 4 wells per plate on the same plate and used as a control for screening mutants with a high GMP conversion ability. The 96-deep well plate inoculated with the control strain and the mutant strains was sealed using Azenta gas permeable seal mark 2, and then incubated for 48 hours at 30°C and 1,000 rpm in a Multitron, an incubator shaker manufactured from Infors-HT. Then, the strains were additionally incubated for 1 hour by adding 1% xylene to the cultured strains and mutant strains, and further incubated at 30°C and 1,000 rpm in a Multitron, an incubator shaker manufactured from Infors-HT, by adding 150 µl of a titer solution containing XMP for 12 hours. The 96-Deep Well Plate incubated for 12 hours was centrifuged for 20 minutes at 15°C and 4,000 rpm using Centrifuge 5810R manufactured by Eppendorf, and 100 µl of the culture supernatant from which the cells were separated was transferred to a 96-Well Black Polystyrene Microplate manufactured by Corning using Biomek i5, a liquid handler manufactured by Beckman Coulter, for NIR spectrometry analysis. Thereafter, the strains were applied to the NIR Spectrometry self-manufactured in the Analysis & Quality Department of CJ CheilJedang Bio Technology Research Institute to secure individual analysis spectrum for each well, and 64 out of 11,451 mutants were primarily selected by applying a regression analysis prediction model with a coefficient of determination of 0.96 for the GMP concentration range of 0 to 20 g/L established based on culture samples whose GMP concentration was quantified through prior HPLC analysis and a selection logic, in which the GMP concentration was improved by 15% or more compared to the control. The 64 selected strains were incubated in the same manner as described above, and the top 5 strains having high GMP concentration were finally selected.

The thus-obtained mutant strains by the above method were named *Corynebacterium ammoniagenes* CJG0497, *Corynebacterium ammoniagenes* CJG0498, *Corynebacterium ammoniagenes* CJG0499, *Corynebacterium ammoniagenes* CJG0500, and *Corynebacterium ammoniagenes* CJG0501, respectively, and among them, the *Corynebacterium ammoniagenes* CJG0497 was deposited at the Korean Culture Center of Microorganisms (KCCM), an International Depositary Authority, under the Budapest Treaty on January 10, 2023 with Accession No. KCCM13320P.

The compositions of the media used in Example 1 and Example 2 are as follows:

### <Seed Medium>

glucose 30 g/L, peptone 15 g/L, yeast extract 15 g/L, sodium chloride 2.5 g/L, urea 3 g/L, adenine 150 mg/L, guanine 150 mg/L, agar 20g/L, pH 7.0 (based on 1 L of distilled water)

### <Titer Evaluation Solution>

Trizma-base 24.20 g/L, ATP 30 g/L, XMP · 2Na · 7H₂O 30 g/L, magnesium sulfate 15 g/L, ammonium sulfate 20g/L

### Example 2: Investigation of Conversion Ability of GMP-Producing Mutant Strains to Convert XMP to GMP

In order to confirm the GMP conversion ability of the *Corynebacterium ammoniagenes* CJG0497, CJG0498, CJG0499, CJG0500, and CJG0501 obtained in Example 1 to convert XMP to GMP, the strains were incubated in the following manner.

The *Corynebacterium ammoniagenes* ATCC6872, the parent strain, and the five mutant strains were each inoculated into a 250 ml corner-baffle flask containing 25 ml of the seed medium, and then incubated with shaking at 30°C and 200 rpm for 20 hours. GMP was prepared by adding 800 µl of the titer evaluation solution to 200 µl of the culture solution and reacting at 42°C for 30 minutes. After completion of the culture, the production amount of GMP was measured using liquid high-performance chromatography, and the GMP concentration in the culture solution for each strain tested is shown in Table 1 below.

**[Table 1]**

| Comparison of GMP Conversion Ability of *Corynebacterium ammoniagenes* ATCC6872, CJG0497, CJG0498, CJG0499, CJG0500, and CJG0501 | | | |
|---|---|---|---|
| Strain No. | XMP Concentratio n (g/l) | GMP Concentration (g/l) | Conversion Rate (%) (GMP produced/XMP consumed) |
| ATCC6872 (Parent strain) | 9.06 | 0.58 | 61.7% |
| CJG0497 (Mutant 1) | 5.63 | 3.45 | 78.9% |
| CJG0498 (Mutant 2) | 7.48 | 1.69 | 67.1% |
| CJG0499 (Mutant 3) | 6.08 | 2.84 | 72.4% |
| CJG0500 (Mutant 4) | 6.44 | 2.58 | 72.5% |
| CJG0501 (Mutant 5) | 6.03 | 2.81 | 70.8% |

As a result, as shown in Table 1, it was confirmed that the *Corynebacterium ammoniagenes* ATCC6872, the parent strain, produced (converted) GMP at a concentration of 0.58 g/l from XMP, but the *Corynebacterium ammoniagenes* CJG0497, CJG0498, CJG0499, CJG0500, and CJG0501, the mutant strains of the present disclosure, converted GMP at concentrations of 3.45 g/l, 1.69 g/l, 2.84 g/l, 2.58 g/l, and 2.81 g/l, respectively from XMP.

That is, it was confirmed that the *Corynebacterium ammoniagenes* CJG0497 strain of the present disclosure exhibited excellent GMP conversion ability and GMP-producing ability.

### Example 3: Investigation of GMP Degradation of GMP-Producing Mutant Strains

In order to confirm the GMP degradation of the *Corynebacterium ammoniagenes* CJG0497, CJG0498, CJG0499, CJG0500, and CJG0501 obtained in Example 1, the strains were incubated in the following manner.

The *Corynebacterium ammoniagenes* ATCC6872, the parent strain, and the five mutant strains were each inoculated into a 250 ml corner-baffle flask containing 25 ml of the seed medium, and then incubated with shaking at 30°C and 200 rpm for 20 hours. GMP was prepared by adding 500 µl of the GMP degradation evaluation medium to 500 µl of the culture solution and reacting at 30°C for 12 hours. After completion of the culture, the GMP concentration was measured using liquid high-performance chromatography, and the GMP concentration in the culture solution for each strain tested is shown in Table 2 below.

**[Table 2]**

| Comparison of GMP Degradation Amount of *Corynebacterium ammoniagenes* ATCC6872, CJG0497, CJG0498, CJG0499, CJG0500, and CJG0501 | | | |
|---|---|---|---|
| Strain No. | GMP Concentration Before Reaction (g/l) | GMP Concentration After Reaction (g/l) | GMP Degradation Amount (g/l) |
| ATCC6872 (Parent strain) | 9.98 | 6.42 | 3.56 |
| CJG0497 (Mutant 1) | 9.89 | 9.39 | 0.50 |
| CJG0498 (Mutant 2) | 10.01 | 6.63 | 3.38 |
| CJG0499 (Mutant 3) | 9.98 | 7.31 | 2.67 |
| CJG0500 (Mutant 4) | 9.99 | 7.49 | 2.50 |
| CJG0501 (Mutant 5) | 10.2 | 7.39 | 2.81 |

As a result, as shown in Table 2, it was confirmed that the *Corynebacterium ammoniagenes* ATCC6872, the parent strain, degraded 3.56 g/l of GMP, but the *Corynebacterium ammoniagenes* CJG0497, CJG0498, CJG0499, CJG0500, and CJG0501, the mutant strains of the present disclosure, degraded 0.50 g/l, 3.38 g/l, 2.67 g/l, 2.50 g/l, and 2.81 g/l of GMP, respectively.

That is, it was confirmed that the *Corynebacterium ammoniagenes* CJG0497 strain of the present disclosure had a reduced GMP degradation rate compared to the parent strain, and could produce GMP with high efficiency and high yield.

The composition of the medium used in Example 3 is as follows.

### <GMP Degradation Evaluation Medium >

GMP 20 g/L, phytic acid 1.8 g/L, magnesium sulfate 4.8 g/L, nymeen 3mℓ/L, xylene 2%, adenine 100mg/L, Na₂HPO₄ 7.7 g/L

### Example 4: Investigation of XMP Degradation of GMP-Producing Mutant Strains

In order to confirm the XMP degradation of the *Corynebacterium ammoniagenes* CJG0497, CJG0498, CJG0499, CJG0500, and CJG0501 obtained in Example 1, the strains were incubated in the following manner.

The *Corynebacterium ammoniagenes* ATCC6872, the parent strain, and the five mutant strains were each inoculated into a 250 ml corner-baffle flask containing 25 ml of the seed medium, and then incubated with shaking at 30°C and 200 rpm for 20 hours. XMP was prepared by adding 500 µl of the XMP degradation evaluation medium to 500 µl of the culture solution and reacting at 30°C for 12 hours. After completion of the culture, the XMP concentration was measured using liquid high-performance chromatography, and the XMP concentration in the culture solution for each strain tested is shown in Table 3 below.

**[Table 3]**

| Comparison of XMP Degradation Amount of *Corynebacterium ammoniagenes* ATCC6872, CJG0497, CJG0498, CJG0499, CJG0500, and CJG0501 | | | |
|---|---|---|---|
| Strain No. | XMP Concentration Before Reaction (g/l) | XMP Concentration After Reaction (g/l) | XMP Degradation Amount (g/l) |
| ATCC6872 (Parent strain) | 10.05 | 7.52 | 2.53 |
| CJG0497 (Mutant 1) | 9.98 | 8.39 | 1.59 |
| CJG0498 (Mutant 2) | 10.01 | 7.71 | 2.30 |
| CJG0499 (Mutant 3) | 10.01 | 7.94 | 2.07 |
| CJG0500 (Mutant 4) | 9.95 | 8.07 | 1.88 |
| CJG0501 (Mutant 5) | 9.99 | 7.79 | 2.20 |

As a result, as shown in Table 3, it was confirmed that the *Corynebacterium ammoniagenes* ATCC6872, the parent strain, degraded 2.53 g/l of XMP, but the *Corynebacterium ammoniagenes* CJG0497, CJG0498, CJG0499, CJG0500, and CJG0501, the mutant strains of the present disclosure, degraded 1.59 g/l, 2.30 g/l, 2.07 g/l, 1.88 g/l, and 2.20 g/l of XMP, respectively.

That is, it was confirmed that the *Corynebacterium ammoniagenes* CJG0497 strain of the present disclosure had a reduced XMP degradation rate compared to the parent strain, and could produce GMP with high efficiency and high yield.

The composition of the medium used in Example 4 is as follows.

### <XMP Degradation Evaluation Medium>

XMP 20 g/L, phytic acid 1.8 g/L, magnesium sulfate 4.8 g/L, nymeen 3mℓ/L, xylene 2%, adenine 100 mg/L, Na₂HPO₄ 7.7g/L

### Example 5: Genetic Analysis of GMP-Producing Mutant Strains

In order to analyze the genetic differences of the *Corynebacterium ammoniagenes* CJG0497 strain obtained in Example 1, a comparative genome analysis experiment was performed with ATCC6872, a wild-type strain of *Corynebacterium ammoniagenes.*

Specifically, the *Corynebacterium ammoniagenes* CJG0497 and ATCC6872 strains, which were plated on an optimal culture medium and incubated for at least 24 hours, were used to extract the genomic DNA (gDNA) for next generation sequencing (NGS), and the cells obtained using a 10-µL loop (SPL inc., Cat. No: 90010) were suspended in 300-µL of PBS. After stirring, 175-µL of the strain suspension was taken, and mixed with Lysis/Binding Solution (mixture of 232-µL of Lysis/Binding Solution Concentrate and 3-µL of Carrier NA; component in MagMaxTM Total Nucleic Acid Isolation Kit) and lysed twice at 6500 rpm for 1 minute in a bead tube. The subsequent process was performed using MagMaxTM Total Nucleic Acid Isolation Kit (Thermo Fisher Scientific Inc., Cat. No. AM1840) according to the method recommended by the manufacturer. The thus-obtained gDNA was quantitatively analyzed using Qubit Fluorometer (Thermo Fisher Scientific Inc.).

The gDNA of the strain obtained for long-read sequencing was diluted to 1500-ng gDNA/48-µL D.W., and Ligation Sequencing Kit (Oxford Nanopore Technologies Inc., Cat. No: SQK-LSK109) and Native Barcoding Expansion 1-12 (PCR-free) Kit (Oxford Nanopore Technologies Inc., Cat. No: EXP-NBD104) were employed for sequencing. In addition, sequencing library was prepared by performing nick and gap filling, dA tailing and barcoding, and adapter ligation processes according to the methods recommended by the manufacturers. The prepared library sample was subjected to sequence analysis for about 24 hours using MinION Mk1C (Oxford Nanopore Technologies Inc.).

The gDNA of the strain obtained for short-read sequencing was diluted to 100-ng gDNA/50-µL D.W., and DNA shearing and barcoding were performed using Ion Plus Fragment Library Kit for Library BuilderTM (Thermo Fisher Scientific Inc., Cat. No: 4477597), Ion XpressTM Plus Fragment Library (Thermo Fisher Scientific Inc., Cat. No: 4477597), and AB Library BuilderTM (Thermo Fisher Scientific Inc.) according to the methods recommended by the manufacturers. Similarly, adapter ligation, nick-repair, and library amplification processes, *etc.* were performed according to the methods recommended by the manufacturers. The finally-obtained sequencing library was diluted to a final concentration of 50-pM, and emulsion PCR enrichment was performed by PC Ion 510^{™} & Ion 520^{™} & Ion 530^{™} Kit - Chef (Thermo Fisher Scientific Inc., Cat. No: A34461) and Ion Chef System (Thermo Fisher Scientific Inc). Finally, the samples were loaded onto Ion 530^{™} Chip (Ion 530^{™} Chip Kit, Thermo Fisher Scientific Inc., Cat. No: A27764) for sequence analysis. After sequence analysis, adapter sequence removal and quality check were performed by Torrent Server, and full-length genome assembly and polishing were performed using the raw sequence data (raw fastq data) obtained therefrom.

**[Table 4]**

| Comparative Analysis of Differences in Gene Sequences of *Corynebacterium ammoniagenes* ATCC6872 and CJG0497 | | | | | |
|---|---|---|---|---|---|
| Gene | Protein | ATCC6872 | | CJG0497 | |
| | | Nucleotide | Peptide | Nucleotide | Peptide |
| hrpB | hypothetical protein | G979 | P327 | A979 | S327 |
| sraP | hypothetical protein | G263 | S88 | A263 | F88 |

**[Table 5]**

| Comparative Analysis of Differences in Non-Coding Region Sequences of *Corynebacterium ammoniagenes* ATCC6872 and CJG0497 | | | | |
|---|---|---|---|---|
| Type | Position | Position Information | Nucleotide | |
| | | | ATCC6872 | CJG0497 |
| rRNA | 1546059 | ORF Name: rrna02 | G | A |
| rRNA | 1546191 | ORF Name: rrna02 | A | G |

As a result, as shown in Table 4, it was confirmed that non-synonym mutations of two genes (hrpB(P327S) and sraP(S88F) were observed in the *Corynebacterium ammoniagenes* CJG0497, the mutant strain of the present disclosure, compared to the *Corynebacterium ammoniagenes* ATCC6872, the parent strain.

In addition, as shown in Table 5, it was confirmed that nucleotide mutations at two rRNA ORF positions were observed in the *Corynebacterium ammoniagenes* CJG0497, the mutant strain of the present disclosure, compared to the *Corynebacterium ammoniagenes* ATCC6872, the parent strain.

**That** is, it was confirmed that the *Corynebacterium ammoniagenes* mutant strain of the present disclosure had genetic differences from the parent strain.

From the foregoing, a skilled person in the art to which the present disclosure pertains will be able to understand that the present disclosure may be embodied in other specific forms without modifying the technical concepts or essential characteristics of the present disclosure. **In** this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium ammoniagenes CJG0497 | Accession number given by the
INTERNATIONAL DEPOSITARY AUTHORITY:
KCCM13320P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on January. 10. 2023 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: January. 10. 2023.

¹ Where Rule 6.4(d) applies, such date is the date on which the status of international depositary authority was
acquired.

Form BP/4 (sole page)

한국미생물보존센터
03641 서울특별시 서대문구 홍제내2가길 45 유림빌딩 Tel: 02-391-0950, 396-0950 Fax: 02-392-2859
KOREAN CULTURE CENTER OF MICROORGANISMS
Yurim Bldg., 45, Hongjenae-2ga-gil, Seodaemun-gu, Seoul, 03641, Korea Tel: 82-2-391-0950, 396-0950 Fax: 82-2-392-2859

## Claims

1. *A Corynebacterium ammoniagenes* CJG0497 strain deposited with Accession No. KCCM13320P.

2. The strain of claim 1, wherein the strain has a 5'-guanosine monophosphate (GMP)-producing ability.

3. The strain of claim 1, wherein the strain has a conversion ability to convert from 5'-xanthosine monophosphate (XMP) to 5'-guanosine monophosphate (GMP).

4. A method for producing 5'-guanosine monophosphate (GMP), comprising culturing a *Corynebacterium ammoniagenes* CJG0497 strain deposited with Accession No. KCCM13320P in a medium.

5. The method of claim 4, further comprising recovering 5'-guanosine monophosphate (GMP) from the cultured strain, a culture product of the strain, a fermented product of the strain, or the culture medium.

6. A composition for producing 5'-guanosine monophosphate (GMP), comprising *a Corynebacterium ammoniagenes* CJG0497 strain deposited with Accession No. KCCM13320P, a culture product of the strain, a fermented product of the strain, or a combination of two or more thereof.

7. Use of a *Corynebacterium ammoniagenes* CJG0497 strain deposited with Accession No. KCCM13320P for the production of 5'-guanosine monophosphate (GMP).
